# EUROPEAN PATENT APPLICATION

(11) **EP 2 586 449 A2**
(43) Date of publication of application: **01.05.2013**
(21) Application number: 11759760.9
(22) Date of filing: 25.03.2011
(51) Int. Cl.: A61K 35/56, A61P 29/00

(54) **LIPID EMULSION HAVING KRILL OIL AS AN ACTIVE INGREDIENT AND PREPARATION METHOD THEREFOR**

(30) Priority: 25.03.2010 KR 20100026837
(71) Applicant: SNU R & DB Foundation, Gwanak-gu, Seoul 151-015 (KR)
(72) Inventor: SHIN, Wan Gyoon, Seoul 110-030 (KR)
(74) Representative: Zeitler - Volpert - Kandlbinder
(86) International application number: PCT/KR2011/002096
(87) International publication number: WO 2011/119011

(57) **Abstract**

Provided is a lipid emulsion having krill oil as an active ingredient and a preparation method therefor, wherein the lipid emulsion can be used independently for oral/parenteral administration or as a daily nutrient, and preferably, as a nutritious intravenous lipid emulsion having krill oil.

## Description

### Technical Field

The present invention relates to a lipid emulsion having krill oil as an active ingredient and a preparation method therefor.

### Background Art

In general, a lipid emulsion is used to meet body lipid demand and/or lipid balance and can be used for oral/parenteral administration. For example, the lipid emulsion may be used to supply a small amount of an isotonic solution with a large amount of energy. When insufficient energy is supplied or voluntary energy supply is not available, the lipid emulsion basically supplies high energy in the form of enteral nutrition or parenteral nutrition to inhibit degeneration of internal protein and supplies essential fatty acids that are not endogenously synthesized in the body.

However, due to a necessity of differentiated energy supply depending on patient's energy metabolism and balance maintenance of body lipids, including cholesterol, research into soybean oil, medium chain triglyceride (MCT) or olive oil used alone or in combination as energy sources is underway, and methods for preparing structured lipids obtained by interesterification of these materials have also been introduced.

In addition, in order to provide polyunsaturated fatty acid (PUFA) including omega 3 fatty acids having anti-inflammatory and immonomodulating capability, fish oil may sometimes be used. However, since fish oil is liable to lipid peroxidation due to PUFA, PUFA is used in a very low content, making it difficult to achieve immunomodulation.

Accordingly, in efforts to develop lipid emulsions capable of reducing the risk of lipid peroxidation while using PUFA in a high content for the purposes of modulating antiflammatory and immune responses and supplying high-calorie energy into the body, the present inventors made intensive researches into the lipid emulsions and developed new lipid emulsion components, thereby completing the present invention.

The lipid emulsion according to the present invention can be used independently for oral/parenteral administration or as a daily nutrient, and as a nutritious intravenous lipid emulsion having krill oil.

The present invention is aiming to provide a new lipid emulsion, which can regulate anti-inflammatory and immune responses in the body while preventing lipid peroxidation, unlike the conventional lipid emulsion.

### Disclosure of the Invention

In order to overcome the above-mentioned shortcomings, the present invention provides a lipid emulsion including as an active ingredient krill oil extracted from krill shrimp, which is a kind of animal planktons.

The present invention also provides a preparation method for a lipid emulsion including krill oil as an active ingredient.

According to an aspect of the invention, there is provided a lipid emulsion having krill oil as an active ingredient.

According to another aspect of the invention, there is provided a preparation method for a lipid emulsion having krill oil as an active ingredient, the preparation method including (a) mixing soybean oil with krill oil extracted from krill shrimp; (b) injecting an emulsifier to the composition of step (a); (c) mixing an isotonic controlling material with the composition of step (b); and (d) sequentially injecting an emulsion stabilizer and distilled water to the composition of step (c) and mixing the same.

According to still another aspect of the invention, there is provided a preparation method for a lipid emulsion having krill oil as an active ingredient, the preparation method including (a) preparing distilled water; (b) mixing an isotonic controlling material with the distilled water; (c) mixing an emulsifier with the composition of step (b); (d) mixing soybean oil and krill oil extracted from krill shrimp with the composition of step (c); and (e) mixing an emulsion stabilizer with the composition of step (d).

### Brief Description of the Drawings

The objects, features and advantages of the present invention will be more apparent from the following detailed description in conjunction with the accompanying drawings, in which:
FIG. 1 shows a certificate for analysis result of astaxanthin contents in krill oil according to embodiments of the present invention;
FIG. 2 shows a certificate for analysis result of components of krill oil according to embodiments of the present invention;
FIG. 3 shows a certificate for analysis result of acid value and peroxide value of krill oil according to embodiments of the present invention;
FIGS. 4 to 19 illustrate graphs and tables of particle size distributions and polydispersity according to the composition ratio of lipid emulsions including krill oil and soybean oil;
FIG. 20 illustrates graphs and tables of particle size distribution and polydispersity of a lipid emulsion including 100% krill oil of 10 weight percent in the lipid emulsion; and
FIGS. 21 to 24 illustrate graphs and tables of particle size distributions and polydispersity according to the composition ratio of lipid emulsions including krill oil and medium chain triglyceride (MCT).

### Best Mode for Carrying Out the Invention

Advantages and features of the present invention and methods of accomplishing the same may be understood more readily by reference to the following detailed description of preferred embodiments and the accompanying drawings. The present invention may, however, be embodied in many different forms and should not be construed as being limited to the embodiments set forth herein. Rather, these embodiments are provided so that this disclosure will be thorough and complete and will fully convey the concept of the invention to those skilled in the art, and the present invention will only be defined by the appended claims. Like numbers refer to like elements throughout.

The terminology used herein is for the purpose of describing particular embodiments only and is not intended to be limiting of the invention. As used herein, the singular forms "a," "an" and "the" are intended to include the plural forms as well, unless the context clearly indicates otherwise. It will be further understood that the terms "comprises" and/or "made of," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Throughout the specification of the present invention, a lipid emulsion means every type of emulsions capable of supplying lipids. Additionally, the lipid emulsion according to the present invention can be mixed or used with carbohydrate or protein sources and additional nutrients such as vitamins or minerals. In addition, the lipid emulsion according to the present invention can be used for all kinds of administration, including both oral and parenteral administration, parenteral nutrition (PN), total parenteral nutrition (TPN), enteral nutrition (EN), total enteral nutrition (TEN) or total nutrient admixture (TNA). The lipid emulsion according to the present invention can be applied for use in food or cosmetics.

The present invention provides a lipid emulsion including krill oil as an active ingredient.

The lipid emulsion according to some embodiments of the present invention may include krill oil extracted from krill shrimp as an active ingredient.

The lipid emulsion according to the present invention may include not only krill oil but also a lipid source, an emulsifier, an emulsion stabilizer and an isotonic controlling material.

For example, the lipid source may include at least one of animal or vegetable oils, such as soybean oil, medium chain triglyceride (MCT), olive oil, sesamic oil, sunflower oil, flax seed oil, cotton seed oil, egg-yolk and fish oil.

Examples of the emulsifier used in embodiments of the present invention may include emulsifiers using phospholipids of egg-yolk, soybean oil or the like. For example, egg-yolk lecithin may be used as the emulsifier.

Examples of the isotonic controlling material used in embodiments of the present invention may include sorbitol, xylitol, or glycerol.

The lipid emulsion according to the embodiment of the present invention may have a particle size of 150 nm to 500 nm and may have high physical stability as intravenous nutrient solution.

An embodiment of the present invention provides a mixed lipid emulsion of krill oil and soybean oil, wherein a mixing ratio of krill oil and soybean oil may be 1:0 to 1:3.

In an embodiment of the present invention, the emulsifier may be included in an amount of about 0.1 wt% to about 50 wt% relative to a total amount of krill oil and soybean oil, for example, in an amount of about 0.5 wt% to about 20 wt%, more specifically, in an amount of about 10 wt%. However, the present invention does not limit the amount of the emulsifier to those listed herein and an appropriate amount may be used to be used as the emulsifier in the lipid emulsion according to the embodiment of the present invention.

Further, the emulsion stabilizer may be included in an appropriate amount to be used to be used as the emulsion stabilizer in the lipid emulsion according to the embodiment of the present invention.

The lipid emulsion according to the present invention may solve the problem of heavy metal contamination of fish oil which has been studied for function of immunomodulation. In addition, the lipid emulsion according to the present invention includes krill oil, which is from a kind of animal planktons, as a new component of lipid emulsion and thereby provides a high functional lipid emulsion

The lipid emulsion according to the present invention can perform essential functions of supplying essential fatty acids, serving as a drug carrier or drug targeting as well as supplying energy high energy in the form of enteral nutrition or parenteral nutrition. In addition, the lipid emulsion according to the present invention can be administered into the body through various routes, including injections, oral administration, nasal administration, eye membrane administration and so on.

The following examples are provided to illustrate further and to facilitate the understanding of the present teachings and are not intended to limit the scope of the present invention. The contents not described herein can be readily envisioned by those skilled in the art and detailed descriptions will not be given. However, the following experimental examples are provided for illustrative purposes and aspects of the present invention are not limited thereto.

### <Experimental Examples>

### Preparation of krill oil

Krill oil was provided from Insung Co., Ltd. of Korea. To extract krill oil, a general extraction method may be used, including organic solvent extraction, supercritical extraction, etc. For example, an extraction method using an organic solvent such as hexane or ethanol, as disclosed in U.S. Patent No.4,331,695, an antioxidant treatment method, as disclosed in U.S. Patent No.5,006,281, or an extraction method disclosed in Canadian Patent No. 1,098,900, may be used to extract krill oil. In the exemplary embodiments of the present invention, the supercritical extraction method was used.

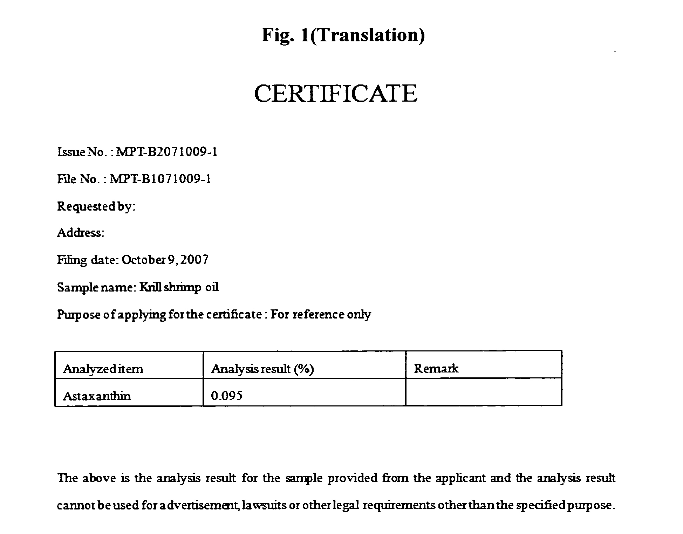

The krill oil used in the embodiments of the present invention may include astaxanthin in an amount of about 0.00001 to about 0.15%. In the exemplary embodiments, the krill oil included astaxanthin in an amount of about 0.095%. FIG. 1 shows a certificate for the contents of astaxanthin included in the krill oil used in the embodiments of the present invention.

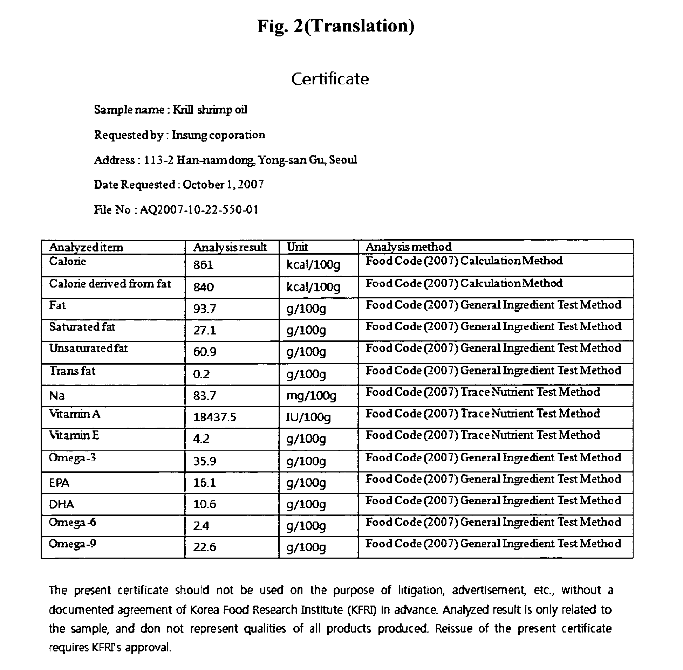

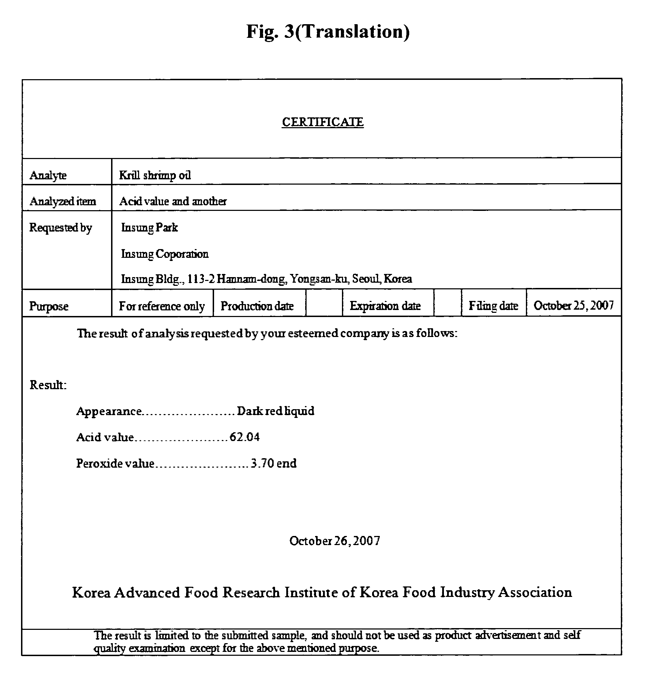

The krill oil may include about 30 to 90 wt%, more specifically in an amount of about 60 wt%, of unsaturated fatty acids. Among the unsaturated fatty acids, omega-3 may be included in the krill oil in an amount of about 40 to 70%, more specifically in an amount of about 58%. FIGS. 2 and 3 show certificates for analysis result of components and acid value and peroxide value of krill oil according to embodiments of the present invention.

### Preparation of Lipid Emulsion I

Krill shrimp oil and soybean oil were mixed in ratios listed in Table 1 below.

**<Table 1>**

| (Content) | Experimental Example 1 (1:0) | Experimental Example 2 (3:1) | Experimental Example 3 (1:1) | Experimental Example 4 (1:3) |
|---|---|---|---|---|
| Krill oil (mg) | 1000 | 750 | 500 | 250 |
| Soybean oil (mg) | 0 | 250 | 500 | 750 |

270 mg of purified egg-yolk lecithin (including greater than 95% of phospholipid and manufactured by Doosan in Manufacture No. GP-90901) was weighed and injected into a conical tube. The krill oil and soybean oil were mixed in ratios of Experimental Examples I to 4 in Table 1 and injected into the conical tube. 220 mg of glycerin was weighed using a micropipette and mixed. 10 mg of oleic acid was weighed using a micropipette and injected into the conical tube. Distilled and deionized water (DDW) was added to the resultant mixture to adjust a final volume to 10 ml. Accordingly, the amount of the mixture including krill oil and soybean oil was in an amount of 10% based on the total weight of the lipid emulsion.

The mixture was stirred at 1000 rpm for 5 minutes using a stirrer (EZ stir TM, High Torque stirrer, HT120AX). Thereafter, the resultant product was homogenized at 8000 rpm for 3 minutes using a homogenizer (JANKE & KUNKEL, IKA, Ultra-Turrax T25). Then, the conical tube was put into an ice bucket and then into an ultrasonic pulverizer (Dr. hielscher sonicator) to be subjected to pulverizing for one minute and cooling for 4 minutes, and this process was repeatedly performed 5 times.

### Measurement of Particle Size of Lipid Emulsion I

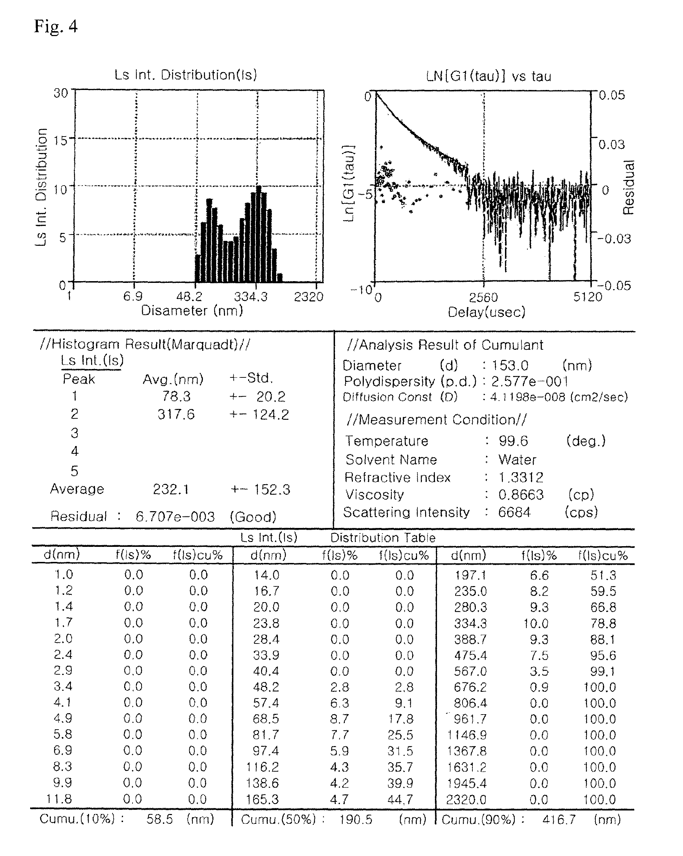

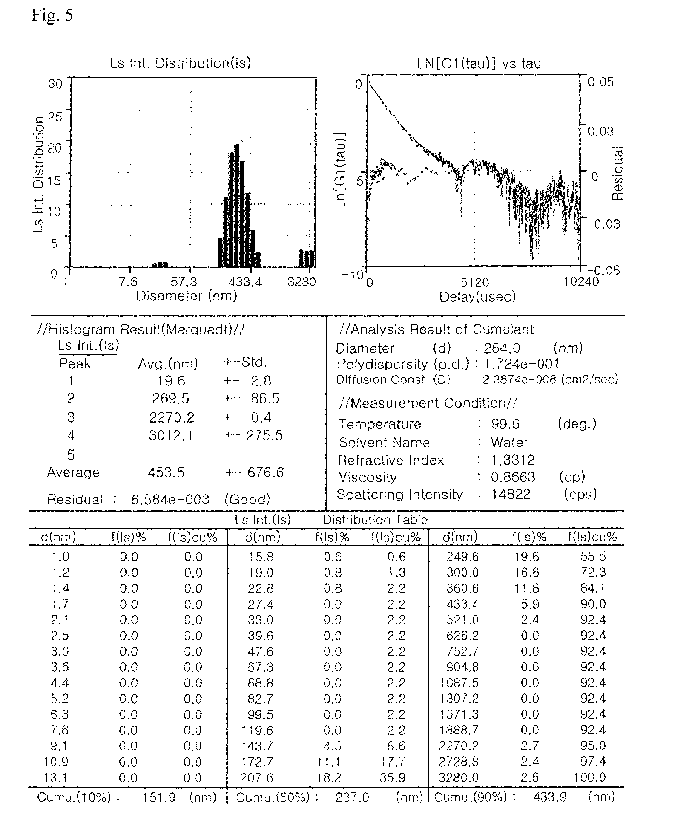

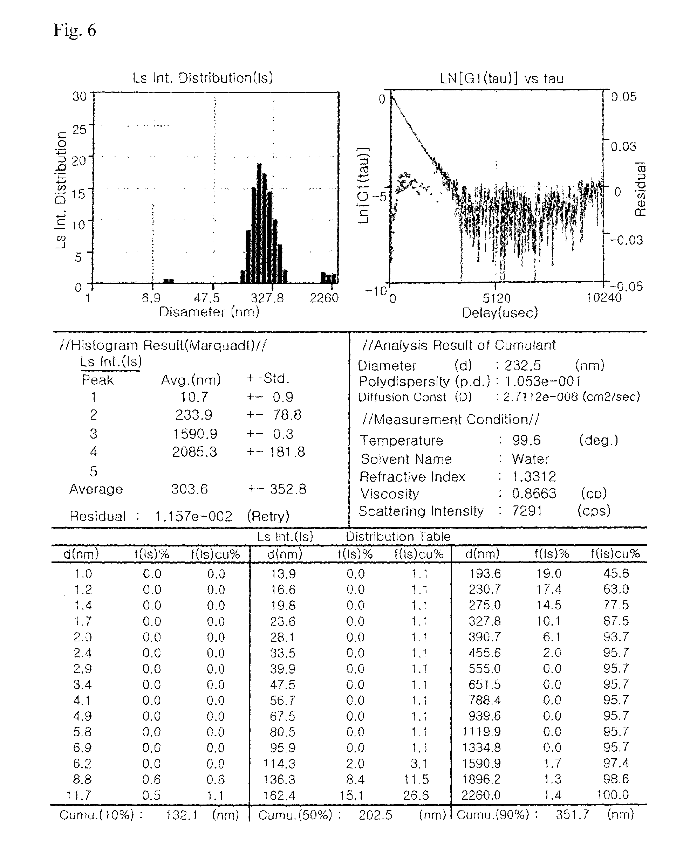

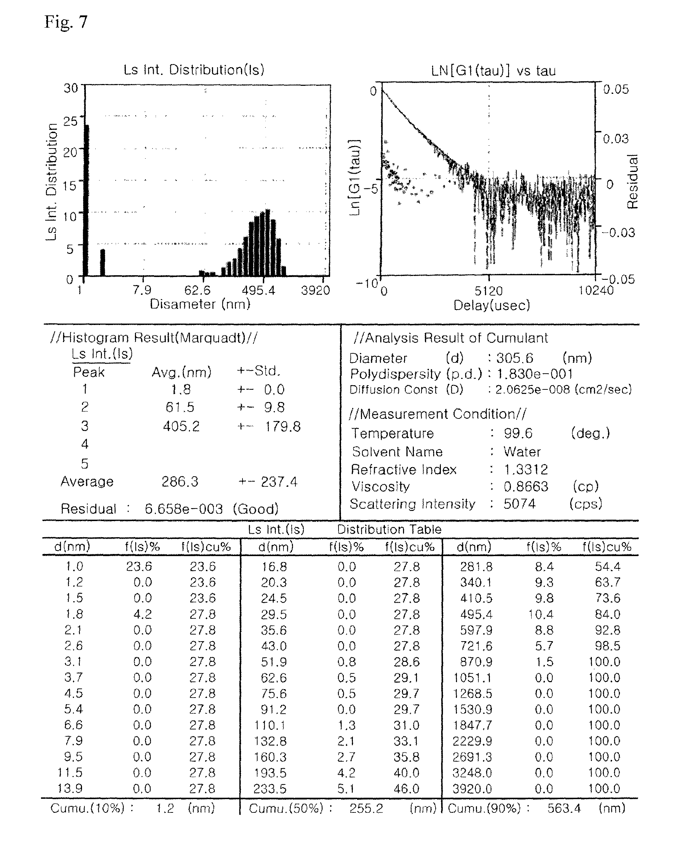

The acquired lipid emulsions were diluted with DDW, the particle size and polydispersity of each of the lipid emulsions were measured using a light scattering method, and the measurement results are shown in FIGS. 4 to 7.

### Preparation of Lipid Emulsion II

Krill shrimp oil and soybean oil were mixed in ratios listed in Table 2 below.

**<Table 2>**

| (Content) | Experimental Example 5 (1:0) | Experimental Example 6 (3:1) | Experimental Example 7(1:1) |
|---|---|---|---|
| Krill oil (mg) | 10000 | 7500 | 5000 |
| Soybean oil (mg) | 0 | 2500 | 5000 |

100 ml of DDW (Daejung Chemical) was put into a beaker to be weighed, 2.2 g of glycerin was added to the beaker to be mixed and 2.7 g of the purified egg-yolk lecithin (Doosan) was added thereto. Thereafter, krill oil and soybean oil were mixed in ratios of Experimental Examples 5 to 7 in Table 2 to then be injected into the beaker. Next, 0.1 g of oleic acid was weighed and added to the beaker. Accordingly, the amount of the mixture including krill oil and soybean oil was 10% based on the total weight of the lipid emulsion.

The mixture was stirred at 8000 rpm for 4 minutes and at 13500 rpm for 6 minutes using a homogenizer. Then, the resultant product was passed in a 140/10 bar state once for 2.5 minutes and in a 540/60 bar state twice using a high-pressure homogenizer (APV homogenizer manufactured by ABB motors).

### Measurement of Particle Size of Lipid Emulsion II

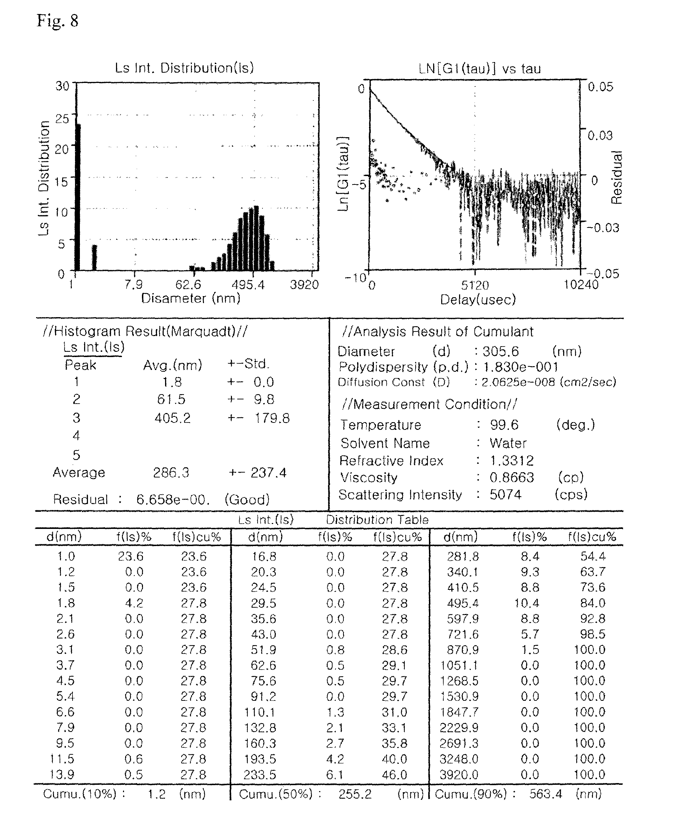

The acquired lipid emulsions were diluted with DDW and the particle size and polydispersity of each of the lipid emulsions were measured using a light scattering method, and the measurement results are shown in FIGS. 8 to 13.

FIGS. 8 and 9 illustrate graphs and tables of particle size distributions and polydispersity according to the composition ratio of a lipid emulsion prepared in Experimental Example 5.

FIGS. 10 and 11 illustrate graphs and tables of particle size distributions and polydispersity according to the composition ratio of a lipid emulsion prepared in Experimental Example 6.

FIGS. 12 and 13 illustrate graphs and tables of particle size distributions and polydispersity according to the composition ratio of a lipid emulsion prepared in Experimental Example 7.

As shown in FIGS. 8 to 13, it was confirmed that the lipid emulsions prepared in Examples of the present invention including krill oil as an active ingredient had a particle size of about 200 nm and demonstrated high physical stability as an intravenous nutrient solution.

### Preparation of Lipid Emulsion III

In Experimental Examples 1 to 4, krill shrimp oil and soybean oil were mixed in ratios listed in Table I and lecithin and oleic acid were mixed in ratios listed in Table 3.

More specifically, 100 ml of DDW (Daejung Chemical) was put into a conical tube to be weighed, 220 mg of glycerin was added to the conical tube to be mixed and a predetermined amount of the purified egg-yolk lecithin (Doosan) was added thereto. Thereafter, krill oil and soybean oil were mixed in such manners as in Experimental Examples I to 4 of Table I to then be injected into the conical tube. Next, a predetermined amount of oleic acid was weighed and added to the conical tube. Accordingly, the amount of the mixture including krill oil and soybean oil was 10% based on the total weight of the lipid emulsion.

The mixture was stirred at 8000 rpm for 4 minutes and at 13500 rpm for 6 minutes using a homogenizer. Then, the resultant product was passed in a 140/10 bar state once for 2.5 minutes and in a 540/60 bar state twice using a high-pressure homogenizer (APV homogenizer manufactured by ABB motors).

**<Table 3>**

| (Content) | Experimental Example 1 (1:0) | Experimental Example 2 (3:1) | Experimental Example 3 (1:1) | Experimental Example 4 (1:3) |
|---|---|---|---|---|
| 270 mg Lecithin | Experimental | Experimental | Experimental | Experimental |
| 30 mg Oleic acid | Example 11 | Example 21 | Example 31 | Example 41 |
| 270 mg Lecithin | Experimental | Experimental | Experimental | Experimental |
| 10 mg Oleic acid | Example 12 (Refer to FIG. 4.) | Example 22 (Refer to FIG. 5.) | Example 32 (Refer to FIG. 6.) | Example 42 (Refer to FIG. 7.) |
| 180 mg Lecithin | Experimental | Experimental | Experimental | Experimental |
| 30 mg Oleic acid | Example 13 | Example 23 | Example 33 | Example 43 |
| 180 mg Lecithin | Experimental | Experimental | Experimental | Experimental |
| 10 mg Oleic acid | Example 14 | Example 24 | Example 34 | Example 44 |

### Measurement of Particle Size of Lipid Emulsion III

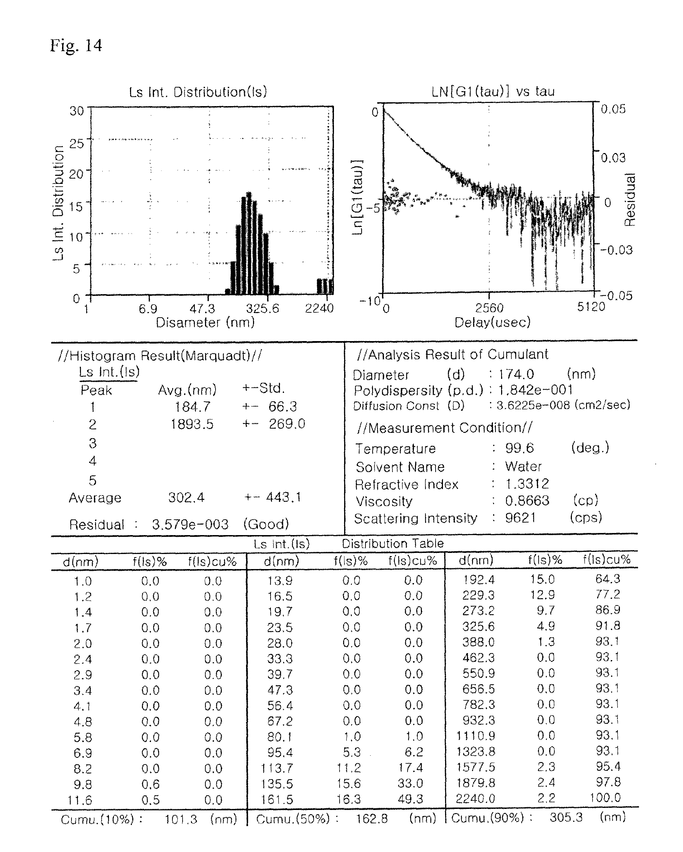

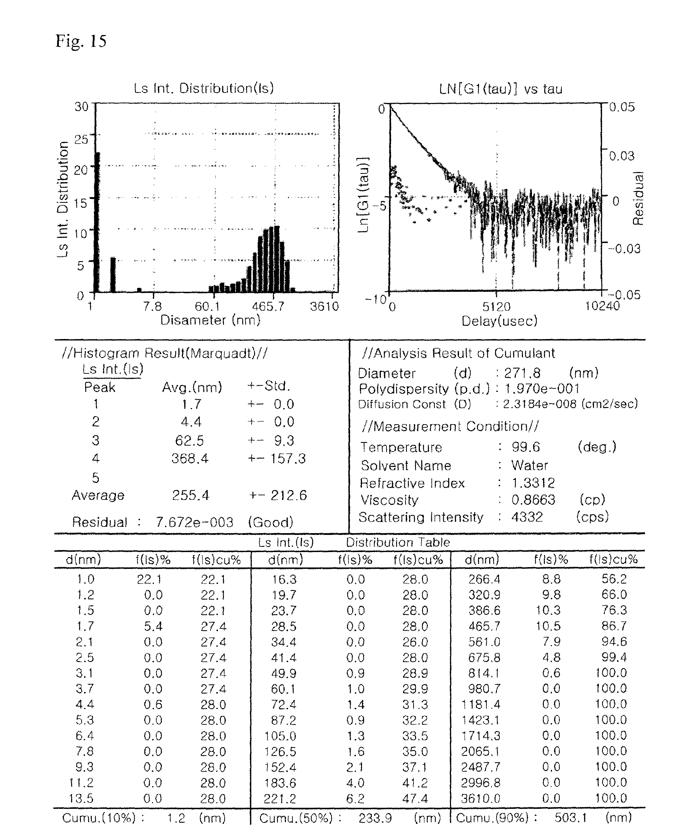

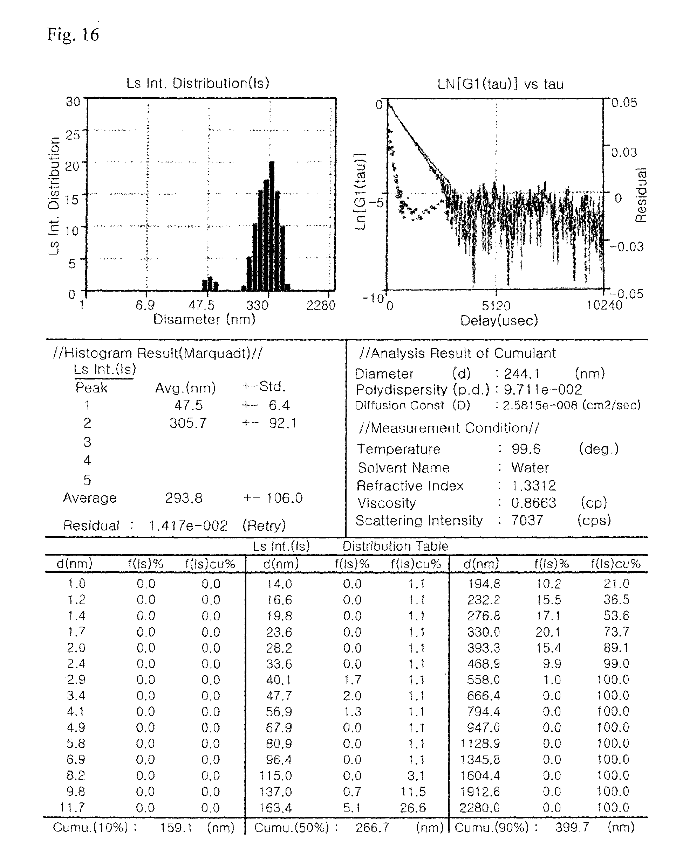

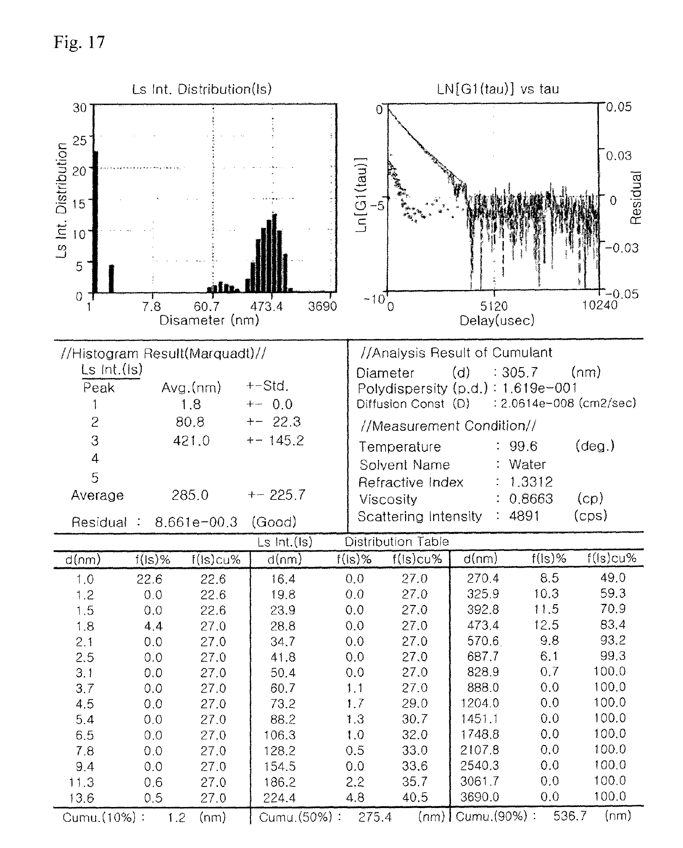

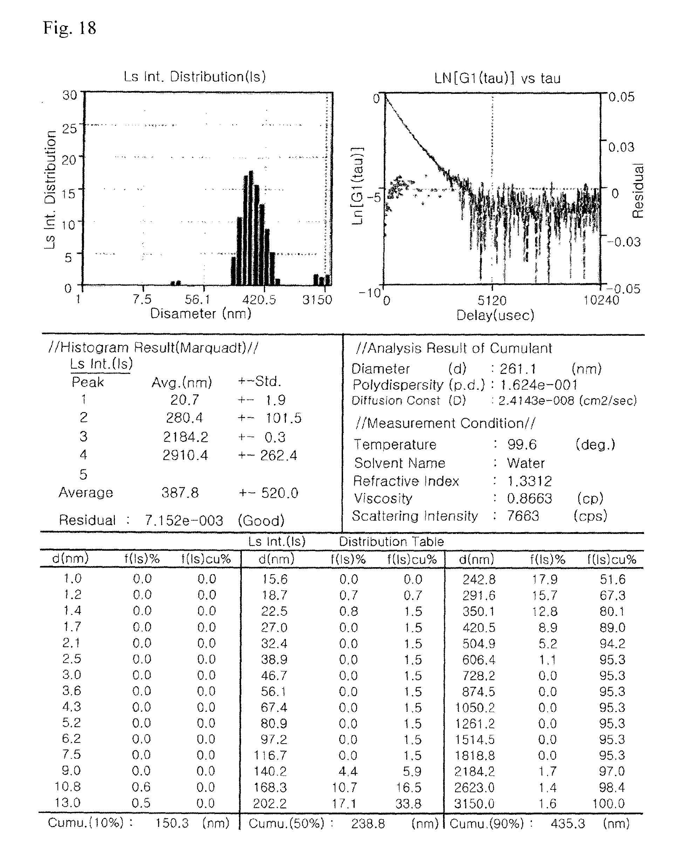

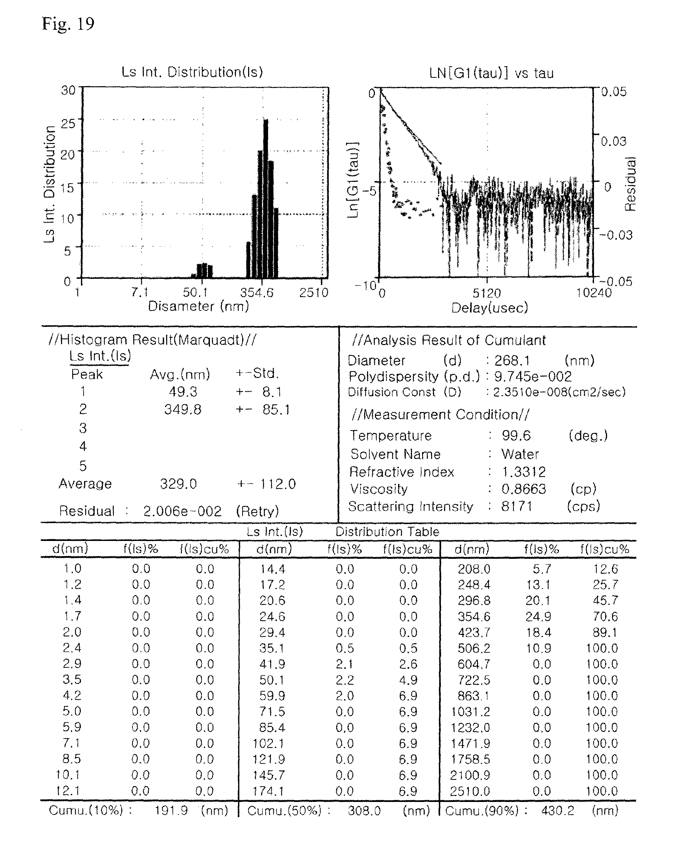

The acquired lipid emulsions listed in Table 3 were diluted with DDW, the particle size and polydispersity of each of the lipid emulsions were measured using a light scattering method, and the measurement results are shown in Table 4. Further, the particle size distributions and polydispersity of the lipid emulsions are shown in FIGS. 14 to 19.

**<Table 4>**

| | | | | |
|---|---|---|---|---|
| (Content) | Experimental | Experimental | Experimental | Experimental |
| | Example 1 (1:0) | Example 2 (3:1) | Example 3 (1:1) | Example 4 (1:3) |
| 270 mg Lecithin | 174.0 nm (Refer to FIG. 14.) | 271.8 nm (Refer to FIG. 15.) | 244.1 nm (Refer to FIG. 16.) | 305.7 nm (Refer to FIG. 17.) |
| 30 mg Oleic acid | | | | |
| 270 mg Lecithin | 153.0 nm (Refer to FIG. 4.) | 264.0 nm (Refer to FIG. 5.) | 232.5 nm (Refer to FIG. 6.) | 305.6 nm (Refer to FIG. 7.) |
| 10 mg Oleic acid | | | | |
| 180 mg Lecithin | NA | NA | 26 1.1 nm (Refer to FIG. 18.) | NA |
| 30 mg Oleic acid | | | | |
| 180 mg Lecithin | NA | NA | 268.1 nm (Refer to FIG. 19.) | NA |
| 10 mg Oleic acid | | | | |

| | | | | |
|---|---|---|---|---|
| * NA: Not Applicable | | | | |

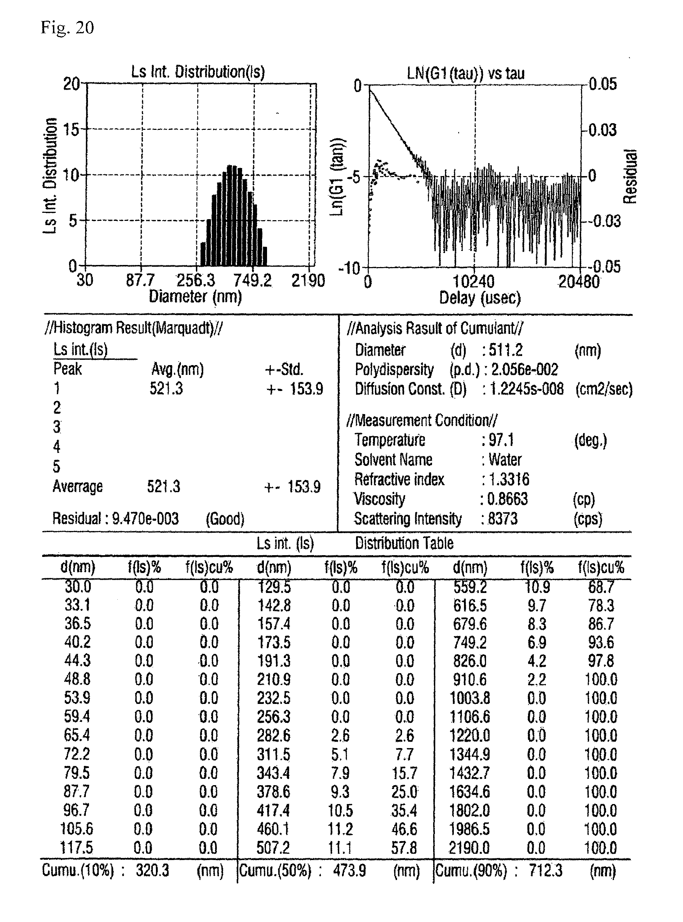

FIG. 20 illustrates the particle size distribution and polydispersity of a lipid emulsion including 100% krill oil of 10 weight percent in the lipid emulsion.

As the Examples described above, according to lipid emulsion having krill oil as an active ingredient and preparation method therefor of the present invention, particles including krill oil and having a suitable size to be used as lipid emulsion can be prepared. In addition, lipid emulsion of the present invention can use krill oil which has low self-contamination, and may further contain omega-3, which is included in krill oil, in a high concentration, astaxanthin having antioxidant effect, and so on.

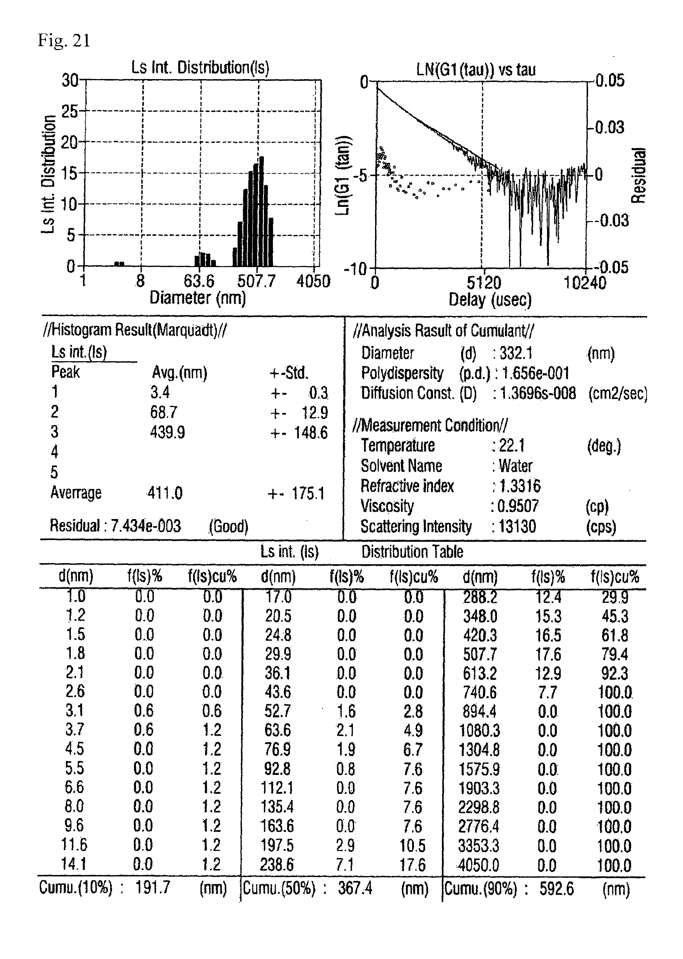

Results of specific experiments performed on lipid emulsions each including krill oil and medium chain triglyceride (MCT) are illustrated in FIGS. 21 to 24. FIGS. 21 to 24 illustrate graphs and tables of particle size distributions and polydispersity according to the composition ratio of lipid emulsions including krill oil and medium chain triglyceride (MCT).

FIG. 21 illustrates a particle size distribution of a lipid emulsion including 10% of a mixture of krill oil and MCT mixed in a ratio of 1:1 based on a total weight of the lipid emulsion and including 300 mg of lecithin and 10 mg of oleic acid.

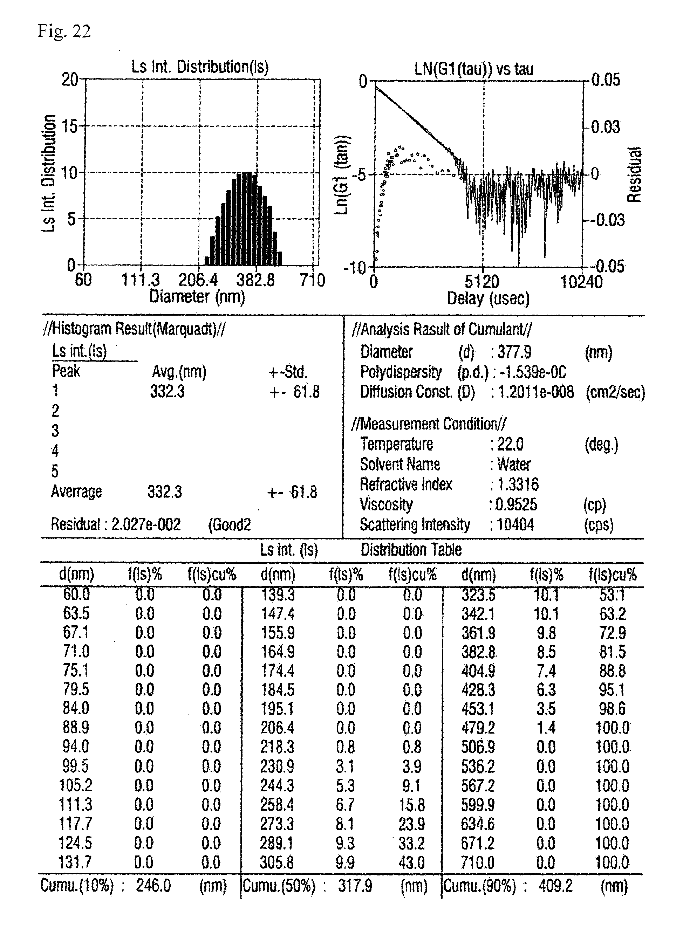

FIG. 22 illustrates a particle size distribution of a lipid emulsion including 20% of a mixture of krill oil and MCT mixed in a ratio of 1:1 based on a total weight of the lipid emulsion and including 300 mg of lecithin and 10 mg of oleic acid.

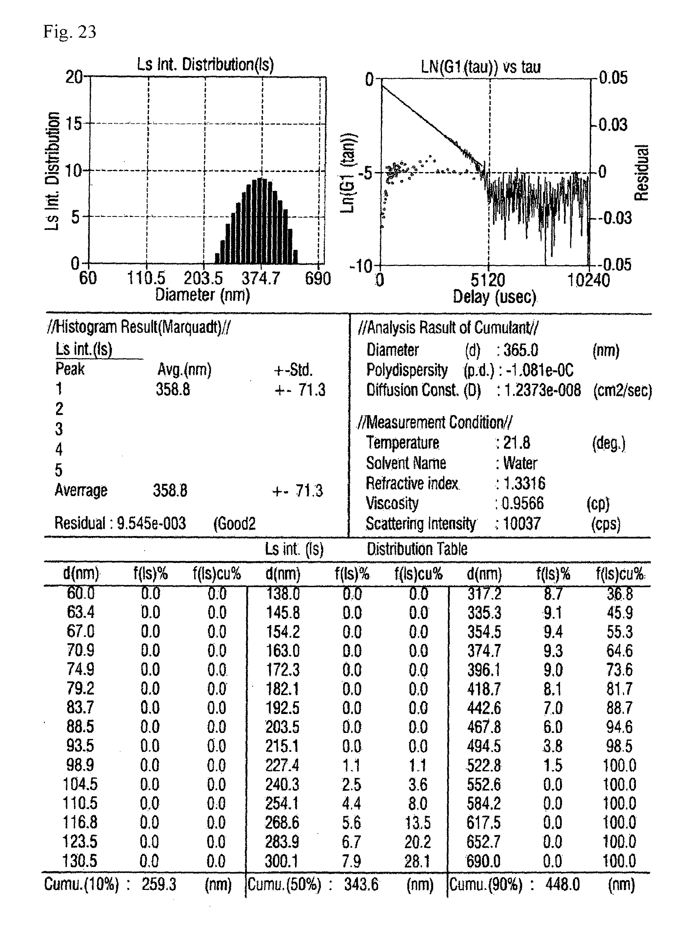

FIG. 23 illustrates a particle size distribution of a lipid emulsion including 20% of a mixture of krill oil and MCT mixed in a ratio of 2:1 based on a total weight of the lipid emulsion and including 300 mg of lecithin and 10 mg of oleic acid.

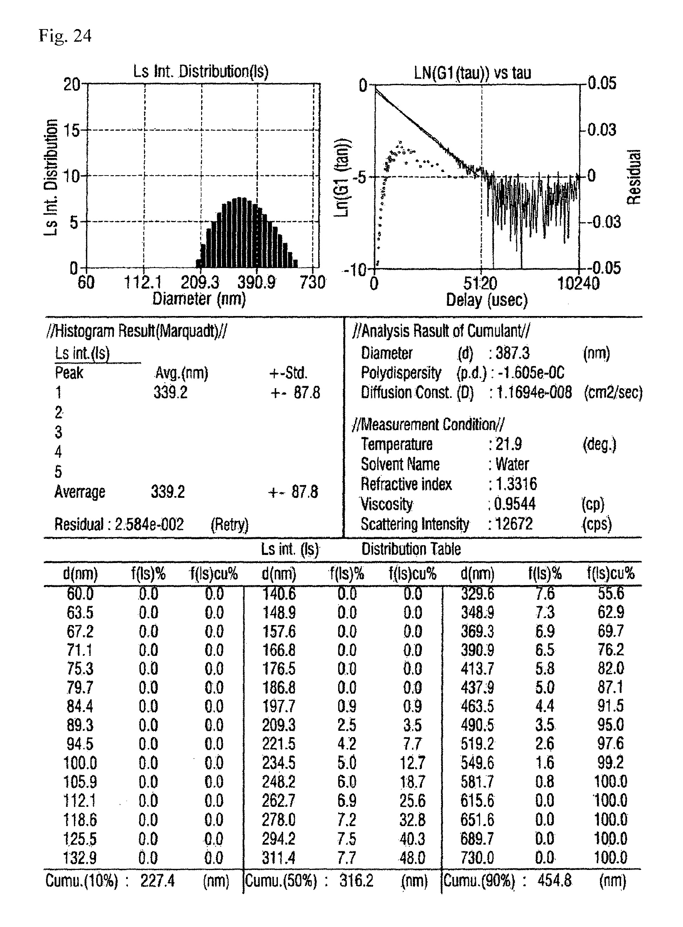

FIG. 24 illustrates a particle size distribution of a lipid emulsion including 20% of a mixture of krill oil and MCT mixed in a ratio of 1:2 based on a total weight of the lipid emulsion and including 300 mg of lecithin and 10 mg of oleic acid.

As described above, According to the embodiments of the present invention, krill oil and soybean oil are dissolved into fine fat particles and can be prevented from being inactivated in the body using the particles as a carrier. Further, the lipid emulsion including the krill oil according to the present invention as an active ingredient is formed into an appropriate particle size to be widely used as a lipid emulsion for intravenous nutrition.

Although exemplary embodiments of the present invention have been described in detail hereinabove, it should be understood that many variations and modifications of the basic inventive concept herein described, which may appear to those skilled in the art, will still fall within the spirit and scope of the exemplary embodiments of the present invention as defined by the appended claims.

## Claims

1. A lipid emulsion comprising krill oil extracted from krill shrimp as an active ingredient.

2. The lipid emulsion of claim 1, further comprising a lipid source, wherein the lipid source is soybean oil.

3. The lipid emulsion of claim 2, wherein the krill oil and the soybean oil are mixed in a ratio of 1:0 to 1:3.

4. The lipid emulsion of claim 2, wherein a mixture of the krill oil and the soybean oil is included in an amount of 5 wt% to 20wt%.

5. The lipid emulsion of claim 2, further comprising an emulsifier and an emulsion stabilizer.

6. The lipid emulsion of claim 5, wherein the emulsifier is included in an amount of about 0.1 wt% to about 50 wt% based on the content of the krill oil and the soybean oil.

7. The lipid emulsion of claim 5, wherein the emulsifier comprises lecithin.

8. The lipid emulsion of claim 5, wherein the emulsion stabilizer includes oleic acid.

9. The lipid emulsion of claim 1, further comprising an isotonic controlling material.

10. The lipid emulsion of claim 9, wherein the isotonic controlling material is glycerin.

11. The lipid emulsion of claim 1, wherein the lipid emulsion has an average particle size of about 200 nm.

12. A preparation method for a lipid emulsion having krill oil as an active ingredient, the preparation method comprising:
(a) mixing soybean oil with krill oil extracted from krill shrimp;
(b) injecting an emulsifier to the composition of step (a);
(c) mixing an isotonic controlling material with the composition of step (b); and
(d) sequentially injecting an emulsion stabilizer and distilled water to the composition of step (c) and mixing the same.

13. The preparation method of claim 12, after step (d), further comprising:
(e) homogenizing the composition of step (d); and
(f) pulverizing and cooling the composition of step (e).

14. The preparation method of claim 13, wherein step (f) is repeatedly performed a number of times.

15. The preparation method of claim 13, wherein in step (f), the pulverizing is performed using a sonicator.

16. The preparation method of claim 12, wherein step (b) comprises injecting lecithin as the emulsifier, step (c) comprises mixing glycerin as the isotonic controlling material with composition of step (b), and step (d) comprises injecting oleic acid as the emulsion stabilizer.

17. A preparation method for a lipid emulsion having krill oil as an active ingredient, the preparation method comprising:
(a) preparing distilled water;
(b) mixing an isotonic controlling material with the distilled water;
(c) mixing an emulsifier with the composition of step (b);
(d) mixing soybean oil and krill oil extracted from krill shrimp with the composition of step (c); and
(e) mixing an emulsion stabilizer with the composition of step (d).

18. The preparation method of claim 17, after step (e), further comprising:
(f) homogenizing the composition of step (e); and
(f) pulverizing and cooling the composition of step (f).

19. The preparation method of claim 18, wherein in step (g), the pulverizing is performed using a high pressure homogenizer.

20. The preparation method of claim 17, wherein step (b) comprises mixing glycerin as the isotonic controlling material with composition of step (b), step (c) comprises injecting lecithin as the emulsifier, and step (e) comprises injecting oleic acid as the emulsion stabilizer.
